# FASCICULE DE BREVET EUROPEEN

(11) **EP 2 285 278 B1**
(45) Date de publication et mention de la délivrance du brevet: **30.01.2013**
(21) Numéro de dépôt: 09750004.5
(22) Date de dépôt: 05.05.2009
(51) Int. Cl.: A61B 5/22, A61H 21/00, A61N 1/05, A61B 5/0488, A61B 5/00, A61N 1/36, A63B 23/20

(54) **DISPOSITIF ET PROCEDE DE MESURE DES SIGNAUX PHYSIOLOGIQUES VAGINAUX ET PERI VAGINAUX ET EN PARTICULIER DU DEBIT SANGUIN ET DES MUSCLES PERI VAGINAUX**
VORRICHTUNG UND VERFAHREN ZUR MESSUNG VAGINALER UND PERIVAGINALER PHYSIOLOGISCHER SIGNALE UND IM BESONDEREN DES BLUTFLUSSES IN DER PERIVAGINALEN MUSKULATUR
DEVICE AND METHOD FOR MEASURING VAGINAL AND PERIVAGINAL PHYSIOLOGICAL SIGNALS AND IN PARTICULAR THE FLOW OF BLOOD IN THE PERIVAGINAL MUSCLES

(30) Priorité: 05.05.2008 FR 0802471
(43) Date de publication de la demande: 23.02.2011
(73) Titulaire: Lavoisier, Pierre, 84110 Rasteau (FR)
(72) Inventeur: Lavoisier, Pierre, 84110 Rasteau (FR)
(74) Mandataire: Maureau, Philippe
(86) Numéro de dépôt international: PCT/FR2009/000522
(87) Numéro de publication internationale: WO 2009/141523

(56) Documents cités:
- WO-A1-2008/124938
- FR-A- 2 774 579
- US-A- 3 709 214
- US-A- 5 702 428
- US-A1- 2003 220 589

## Description

La présente invention est relative à un procédé de mesure permettant de mesurer les pressions et les variations de pressions lors d'une contraction des muscles péri vaginaux, de mesurer les débits artério-veineux, de mesurer la circulation vaginale et clitoridienne, ainsi qu'un dispositif pour la mise en oeuvre de ce procédé.

On connait d'après le brevet américain US 2003/0220589 un dispositif pour la rééducation des muscles du plancher pelvien par l'émission de vibrations entrainant la contraction des muscles. Ce dispositif permet de guérir ou de prévenir l'incontinence urinaire féminine. Le patient peut régler sur le dispositif et pour la contraction de chaque muscle l'intensité et le nombre de contraction ainsi que la durée et la répétition des périodes de contraction.

On connait d'après le brevet français FR 2 774 579 une sonde vaginale pour faire travailler le muscle du périnée. Cette sonde vaginale permet de réaliser en temps réel la mesure de la déformation effective du muscle du périnée. La sonde permet aussi de quantifier la valeur de la déformation effective du muscle du périnée.

Cette sonde comporte des premiers moyens permettant de stimuler le muscle du périnée par l'intermédiaire du courant électrique de fréquence donnée. La sonde comporte des seconds moyens permettant de mesurer la déformation du muscle du périnée sous l'effet de la stimulation électrique produite par le courant basse fréquence.

Le dispositif et le procédé de mesure suivant la présente invention consiste à améliorer les dispositifs connus en permettant notamment de mesurer l'état de fatigue de chaque muscle péri vaginal (PV), de mesurer les variations de la force musculaire de chaque muscle péri vaginal (PV) et de mesurer le module de Young du corps caverneux clitoridien permettant d'évaluer la compliance dudit corps caverneux clitoridien.

Ces mesures réalisées par le dispositif sont effectuées soit au cours du sommeil de la patiente soit le jour et permettent, en fonction des résultats obtenus, de déterminer la cause des dysfonctions et d'en contrôler l'évolution.

La mesure des variations de la tonicité des muscles péri vaginaux au cours du sommeil permet de dissocier les dysfonctions sexuelles organiques des dysfonctions sexuelles psychogènes. Les patientes ayant une dysfonction sexuelle organique n'ont pas de variations du tonus musculaire ou du débit sanguin au cours des phases du sommeil paradoxal.

Également, le dispositif de mesure objet de l'invention permet de suivre l'évolution en mesurant les effets de la rééducation des patientes dans le cas d'incontinence urinaire d'effort ainsi que dans le cas d'anorgasmie.

Lors de la mesure permettant de suivre l'évolution des effets de la rééducation périnéale chez la femme, la mesure de la pression provoquée par les muscles péri vaginaux est parasitée par la contraction abdominale.

En effet, pour améliorer l'amplitude du signal visualisé à l'écran, les patientes ont tendance à contracter non seulement leurs muscles péri vaginaux, ce qui est le but, mais aussi les abdominaux ce qui majore artificiellement le résultat et perturbe complètement les mesures.

Cette contraction abdominale augmente la pression intra vaginale qui augmente la pression sur le dispositif de mesure surtout s'il s'agit d'une sonde gonflable laquelle obture hermétiquement l'orifice vaginal.

Le procédé de mesure et son dispositif pour sa mise en oeuvre ont pour objet de remédier aux inconvénients énoncés ci-dessus.

Le procédé de mesure permettant de mesurer les signaux physiologiques vaginaux et péri vaginaux comme les pressions et les variations de pressions lors d'une contraction des muscles péri vaginaux (PV), mais aussi le pouls péri vaginal et clitoridien, la pression intra caverneuse clitoridienne, la température vaginale mais aussi tout autre signal physiologique pertinent suivant la présente invention consiste à :
- Placer un capteur comportant des moyens pour capter la pression et les variations de pressions lors d'une contraction des muscles péri vaginaux (PV), et au moins un transducteur permettant de transformer les signaux de pression issus des moyens pour capter la pression en signaux électriques,
- Et à connecter le capteur à une carte électronique ou à un boîtier comportant des moyens d'amplification et d'enregistrement du signal provenant des transducteurs appartenant au capteur, des moyens d'analyse des signaux par un microcontrôleur permettant de mesurer l'état de fatigue de chaque muscle péri vaginal (PV), de mesurer les variations de la force musculaire des muscles péri vaginaux (PV) et de mesurer le module de Young du corps caverneux clitoridien permettant d'évaluer la compliance dudit corps caverneux clitoridien.

Le procédé de mesures suivant la présente invention consiste à mesurer les variations de la pression intra caverneuse (PIC) afin de quantifier les variations du tonus de chaque muscle péri vaginal et de leur circulation artérielle et veineuse de jour comme de nuit.

Le procédé de mesure suivant la présente invention consiste à mesurer la pression intra caverneuse (PIC) lorsqu'elle atteint progressivement la pression systolique moyenne.

Le procédé de mesure suivant la présente invention consiste à mesurer pendant la phase vasculaire qui est la première phase de l'érection clitoridienne, l'augmentation du débit artériel d'entrée dans le corps caverneux clitoridien et la diminution du débit veineux de sortie.

Le procédé de mesure suivant la présente invention consiste à mesurer pendant la phase musculaire qui est la seconde phase de l'érection clitoridienne, les variations de la contraction des muscles péri vaginaux (PV), dont les muscles ischio caverneux (IC) entourant le corps caverneux clitoridien.

Le procédé de mesure suivant la présente invention consiste à mesurer en continu le débit artériel d'entrée et le débit veineux de sortie par la mesure de la pression du pouls (PP) et du volume du pouls (VP).

Le procédé de mesure suivant la revendication 1, caractérisé en ce qu'il consiste à mesurer la fuite cavemo veineuse à partir de la courbe de la pression intra caverneuse (PIC).

Le procédé de mesure suivant la présente invention consiste à mesurer la contraction maximum (Pmax), le gradient de pression (Delta P et Delta Moyen), la surface sous la courbe traduisant le travail musculaire de chaque contraction de chaque muscle péri vaginal, le nombre de pics et la largeur de chaque pic.

Le procédé de mesure suivant la présente invention consiste à mesurer la fatigue de chaque muscle péri vaginal (PV) en mesurant l'angle (*α*).

Le procédé de mesure suivant la présente invention consiste à mesurer les pics des contractions de chaque muscle péri vaginal (PV) et les variations de pression du pouls (PP) et de volume du pouls (VP).

Le procédé de mesure pour la mise en oeuvre du procédé suivant la présente invention comprend un capteur qui est constitué d'une sonde comportant un corps longitudinal cylindrique pourvu d'une collerette formant butée, un capteur de pression placé suivant une direction longitudinale à la surface du corps cylindrique, ledit capteur de pression comprenant au moins un transducteur permettant de transformer les signaux de pression issus des moyens pour capter la pression en signaux électriques, une carte électronique disposée à l'intérieur du corps cylindrique et à laquelle est connecté le capteur de pression, et des moyens de communication traversant longitudinalement la sonde qui sont constitués par au moins une cannelure longitudinale ménagée sur la périphérie du corps cylindrique et traversant la collerette pour permettre de relier la partie interne du vagin à l'extérieur afin d'équilibrer les pressions intra vaginales et extra vaginales, ladite carte électronique comportant des moyens d'amplification et d'enregistrement des signaux provenant des transducteurs, des moyens d'analyse des signaux par un microcontrôleur permettant de mesurer l'état de fatigue de chaque muscle péri vaginal (PV), de mesurer les variations de la force musculaire de chaque muscle péri vaginal (PV) et de mesurer le module de Young du corps caverneux clitoridien permettant d'évaluer la compliance dudit corps caverneux clitoridien.

Le dispositif de mesure pour la mise en oeuvre du procédé suivant la présente invention comprend un capteur qui est constitué d'une sonde comportant un corps longitudinal cylindrique pourvu d'une collerette formant butée, un capteur de pression placé suivant une direction longitudinale à la surface du corps cylindrique, ledit capteur de pression comprenant au moins un transducteur permettant de transformer les signaux de pression issus des moyens pour capter la pression en signaux électriques, une carte électronique disposée à l'intérieur du corps cylindrique et à laquelle est connecté le capteur de pression, et des moyens de communication traversant longitudinalement la sonde qui sont constitués par un canal ménagé à l'intérieur du corps cylindrique et traversant longitudinalement ce dernier et la collerette pour permettre de relier la partie interne du vagin à l'extérieur afin d'équilibrer les pressions intra vaginales et extra vaginales, ladite carte électronique comportant des moyens d'amplification et d'enregistrement des signaux provenant du transducteur, des moyens d'analyse des signaux par un microcontrôleur permettant de mesurer l'état de fatigue de chaque muscle péri vaginal (PV), de mesurer les variations de la force musculaire de chaque muscle péri vaginal (PV) et de mesurer le module de Young du corps caverneux clitoridien permettant d'évaluer la compliance dudit corps caverneux clitoridien.

Le dispositif de mesure pour la mise en oeuvre du procédé suivant la présente invention comprend un capteur qui est constitué d'une sonde comportant un corps longitudinal cylindrique pourvu d'une collerette formant butée, un capteur de pression placé suivant une direction longitudinale à la surface du corps cylindrique, ledit capteur de pression comprenant au moins un transducteur permettant de transformer les signaux de pression issus des moyens pour capter la pression en signaux électriques, une carte électronique disposée à l'intérieur du corps cylindrique et à laquelle est connecté le capteur de pression, et des moyens de communication traversant longitudinalement la sonde qui sont constitués d'une part par au moins une cannelure longitudinale ménagée sur la périphérie du corps cylindrique et traversant la collerette et d'autre part par un canal ménagé à l'intérieur du corps cylindrique et traversant longitudinalement ce dernier et la collerette, lesdits moyens de communication permettant de relier la partie interne du vagin à l'extérieur afin d'équilibrer les pressions intra vaginales et extra vaginales, ladite carte électronique comportant des moyens d'amplification et d'enregistrement des signaux provenant du transducteur, des moyens d'analyse des signaux par un microcontrôleur permettant de mesurer l'état de fatigue de chaque muscle péri vaginal (PV), de mesurer les variations de la force musculaire de chaque muscle péri vaginal (PV) et de mesurer le module de Young du corps caverneux clitoridien permettant d'évaluer la compliance dudit corps caverneux clitoridien.

Le dispositif de mesure pour la mise en oeuvre du procédé suivant la présente invention comprend une sonde comportant à la surface du corps cylindrique des lamelles d'électro-stimulation connectées à la carte électronique.

Le dispositif de mesure suivant la présente invention comprend des lamelles d'électro-stimulation positionnées parallèlement à l'axe longitudinal dudit corps cylindrique.

Le dispositif de mesure suivant la présente invention comprend des lamelles d'électro-stimulation présentant un profil circulaire ou sphérique.

Le dispositif de mesure suivant la présente invention comprend un capteur de pression constitué d'une lamelle métallique qui est fixée dans une rainure ménagée dans le corps cylindrique, ladite rainure étant remplie d'un matériau composite élastique permettant d'absorber les mouvements ou les déformations élastiques de ladite lamelle, tandis que la face interne de la lamelle métallique est solidaire d'une jauge de contrainte ou transducteur permettant de transformer le signal de pression issu de la lamelle métallique en un signal électrique.

Le dispositif de mesure suivant la présente invention comprend un capteur de pression constitué de transducteurs formant un film piezzo électrique fixé au niveau de la rainure remplie d'un matériau composite élastique.

Le dispositif de mesure suivant la présente invention comprend une sonde comportant à l'intérieur du corps cylindrique un évidemment permettant la mise en place et la retenue d'une carte électronique.

Le dispositif de mesure suivant la présente invention comprend une sonde comportant tous les capteurs nécessaires aux mesures physiologiques vaginales connectés à la carte électronique.

Le dispositif de mesure suivant la présente invention comprend une carte électronique qui peut être raccordée à un boîtier contenant un ou des amplificateur(s), un convertisseur A/D, et un microprocesseur.

Le dispositif de mesure pour la mise en oeuvre du procédé de mesures suivant la présente invention comprend un capteur qui est constitué :
- D'un anneau ouvert comportant deux branches en arc de cercle reliées entre elles par un élément souple,
- Et d'une jauge de contrainte solidaire de l'élément souple et connectée à un boîtier ou à une carte électronique.

Le dispositif de mesure suivant la présente invention comprend une jauge de contrainte qui est disposée sur la face interne de l'élément souple afin que cette dernière se trouve à l'intérieur de l'anneau ouvert pour éviter tout risque de blessure du vagin de la patiente.

Le dispositif de mesure suivant la présente invention comprend des branches qui sont plates en forme de lamelle et réalisées dans un matériau ayant une grande résistance à la déformation élastique ou dit peu déformable.

Le dispositif de mesure suivant la présente invention comprend un élément souple reliant les branches qui est constitué d'une fine platine de métal présentant des caractéristiques de déformation élastique importante.

Le dispositif de mesure suivant la présente invention comprend un anneau ouvert qui comporte sur la face externe des branches des moyens d'électro stimulation qui sont constitués d'électrodes connectées à un boîtier contenant un amplificateur, un convertisseur A/D, un microprocesseur, une carte électronique et micro contrôleur gérant l'électro stimulation.

Les dessins annexés, donnés à titre d'exemple, permettront de mieux comprendre l'invention, les caractéristiques qu'elle présente et les avantages qu'elle est susceptible de procurer :
Figures 1 et 2 sont des vues de coté illustrant le dispositif de mesure suivant la présente invention.
Figures 3 à 6 sont des vues schématiques en coupe représentant le dispositif de mesure suivant la présente invention.
Figure 7 est un schéma montrant la variation de la pression mesurée par le dispositif de mesure et de surveillance suivant la présente invention.
Figures 8 à 10 sont des vues illustrant un dispositif de mesure comportant un capteur réalisé à partir d'un anneau ouvert suivant la présente invention.

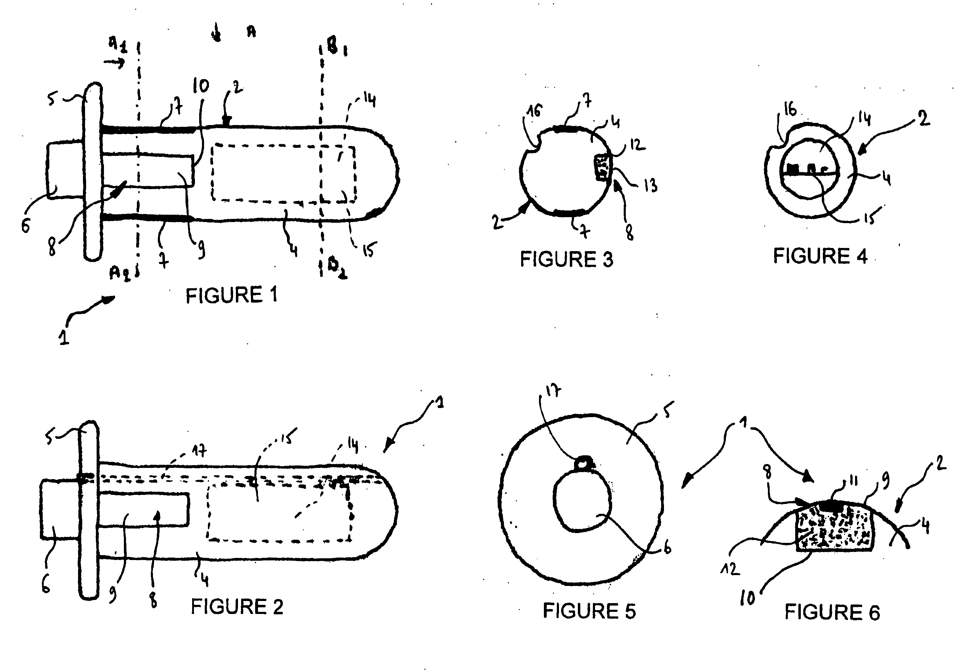

On a montré en figures 1 à 6 un dispositif de mesure 1 permettant de mesurer les pressions et les variations de pressions lors d'une contraction de muscles péri vaginaux (PV).

Le dispositif de mesure 1 comprend un capteur qui est constitué d'une sonde 2 comportant des moyens pour capter la pression et les variations de la pression dues à la force musculaire des muscles péri vaginaux (PV).

La sonde 2 est constituée d'un corps cylindrique 4 rigide réalisé dans un matériau plastique non déformable.

Le corps longitudinal cylindrique 4 est pourvu d'une collerette 5 formant butée et permettant de positionner ladite sonde à l'intérieur du vagin dans une position déterminée et un méplat 6 assurant le maintien et la préhension de ladite sonde.

La collerette 5 est positionnée entre le corps longitudinal cylindrique 4 et le méplat 6. Ce dernier peut être prévu de dimension extérieure identique ou inférieure à celle du corps longitudinal cylindrique 4.

La sonde 2 comporte au voisinage de la collerette 5 et fixées sur le corps cylindrique 4 des lamelles d'électro-stimulation 7 qui peuvent être au nombre de deux ou quatre. Ces dernières sont positionnées à la surface du corps cylindrique 4 et suivant une direction qui est parallèle à l'axe longitudinal dudit corps cylindrique 4.

Les lamelles d'électro-stimulation 7 sont réalisées dans un matériau conducteur qui peut être soit un alliage ou élastomère conducteur. Les lamelles d'électro-stimulation 7 peuvent présenter un profil circulaire ou sphérique ou toute autre forme permettant une bonne électro-stimulation et une bonne mesure de la pression. En effet, les lamelles d'électro-stimulation 7 peuvent servir de capteur de pression.

La sonde 2 comporte des moyens pour capter la pression et les variations de pressions lors d'une contraction des muscles péri vaginaux (PV) qui sont constitués d'un capteur de pression 8 placé suivant une direction longitudinale à la surface du corps cylindrique 4 et entre les lamelles d'électro-stimulation 7.

Le capteur de pression 8 est constitué d'une lamelle métallique 9 qui est fixée dans une rainure 10 ménagée, à partir de la collerette 5, dans le corps cylindrique 4 de la sonde 2.

La lamelle métallique 9 est solidaire sur sa face interne d'une jauge de contrainte 11 ou transducteur permettant de transformer le signal de pression issu de la lamelle métallique 9 en un signal électrique.

Préalablement à la fixation de la lamelle métallique 9, la rainure 10 est remplie d'un matériau composite élastique 12 permettant d'absorber les mouvements ou les déformations élastiques de ladite lamelle 9 (figures 1, 2, 6).

Le capteur de pression 8 peut être constitué d'un film piezzo électrique 13 ou transducteur fixé au niveau de la rainure 10 remplie du matériau composite élastique 12. Dans ce cas le piezzo électrique 13 ne comporte pas de jauge de contrainte, car ce dernier, par sa constitution, permet de transformer le signal de pression issu dudit film en un signal électrique (figure3).

La sonde 2 comporte à l'intérieur du corps cylindrique 4 un évidemment 14 permettant la mise en place et la retenue d'une carte électronique 15 à laquelle sont connectées les lamelles d'électro-stimulation 7 et /ou le ou les capteur(s) de pression 8 ou tout autre capteur ou autres stimulateurs électriques ou mécaniques (figures 1, 2 et 4).

La carte électronique 15 comporte des moyens d'enregistrement et d'amplification du signal provenant de la jauge de contrainte 11 ou du film piezzo électrique 13 formant chacun un transducteur.

La carte électronique 15 comporte des moyens d'analyse du signal par un microcontrôleur permettant de surveiller l'électro-stimulation des lamelles 7 en fonction de l'état de fatigue des muscles péri vaginaux (PV) et de mesurer l'intensité de la force musculaire par l'intermédiaire de gabarits sonores ou visuels ou tactiles indiquant à la patiente l'intensité, la durée de la contraction musculaire à produire et la durée de la période de repos entre deux contractions.

La carte électronique 15 comprend au moins une batterie rechargeable éventuellement par induction assurant l'autonomie nécessaire à la sonde 2 et à son électronique associée.

La sonde 2 comprend sur la périphérie du corps cylindrique 4 au moins une cannelure longitudinale 16 traversant par un conduit la collerette 5 constituant des moyens de communication de pression entre la partie interne du vagin et l'extérieur afin d'équilibrer les pressions intra vaginales et extra vaginales (figures 3 et 4).

Ces moyens de communication peuvent être également réalisés par l'intermédiaire d'au moins un canal 17 ménagé à l'intérieur du corps cylindrique 4 et débouchant d'une part à l'extérieur au-dessus du méplat de préhension 6 et à l'intérieur du vagin au niveau de l'extrémité libre dudit corps cylindrique (figures 2 et 5).

Egalement la sonde 2 peut comporter à la surface du corps cylindrique 4 des capteurs connectés à la carte électronique 15 et mesurant les signaux physiologiques à savoir la pression, la température, la lubrification le débit sanguin en particulier par photoplethysmographie ainsi que tous signaux physiologiques pertinents.

Lorsque la sonde 2 comporte deux lamelles d'électro-stimulation 7, ces dernières peuvent servir en même temps de stimulation électrique et de mesure de la pression, le micro contrôleur gérant un temps de stimulation puis un temps de mesure.

Lorsque la sonde 2 est introduite à l'intérieur du vagin, on constate que la collerette périphérique 5 de la sonde 2 vient en appui contre la vulve permettant de garantir que les lamelles d'électro-stimulation 7 et le capteur de pression 8 soient toujours en contact avec la même partie de la paroi vaginale et plus particulièrement en regard de la musculature péri vaginale (PV) de la patiente.

Les lamelles d'électro-stimulation 7 sont connectées à la carte électronique 15 comprenant un système d'électro-stimulation et permettant d'envoyer de faibles impulsions électriques aux dites lamelles d'électro-stimulation 7 afin d'assurer une électro-stimulation contrôlée des muscles péri vaginaux (PV), tandis que les informations réceptionnées par la carte électronique 15 sont envoyées à un boîtier.

On note que la stimulation électrique des lamelles d'électro-stimulation 7 est activée par la patiente de manière à être bien perçue sans jamais être douloureuse. La patiente peut à tout moment en faire varier l'intensité.

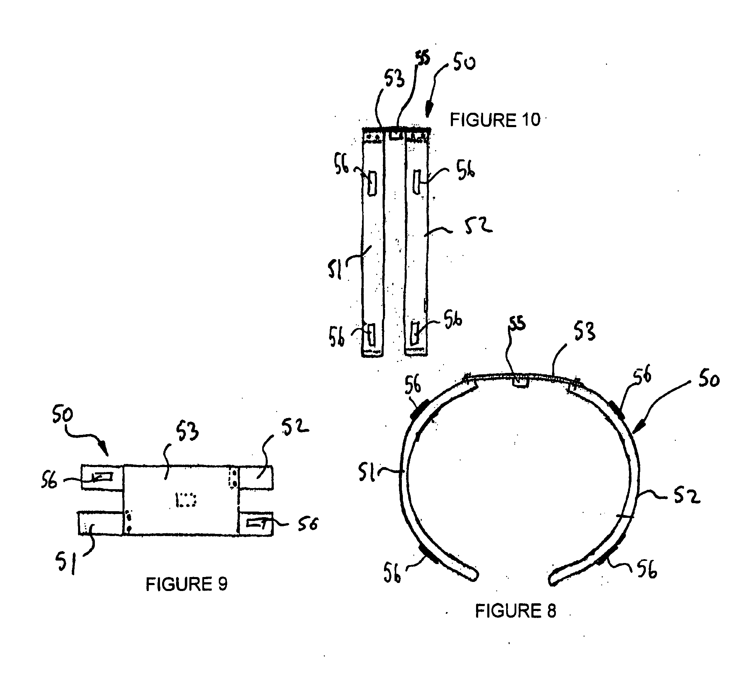

On a illustré en figures 8 à 10 un dispositif de mesure 1 qui est constitué d'un capteur formé d'un anneau ouvert 50 comportant deux branches 51, 52 en arc de cercle reliées entre elles au sommet du profil circulaire par un élément souple 53.

Les branches 51, 52 sont plates en forme de lamelle et réalisées dans un matériau ayant une grande résistance à la déformation élastique ou dite peu déformable.

L'élément souple 53 reliant les branches 51, 52 est constitué d'une fine platine de métal présentant des caractéristiques de déformation élastique importante. L'élément souple 53 comporte sur l'une de ses faces une jauge de contrainte 55 qui est connectée par tout moyen soit à une carte électronique, soit à un boîtier, non représenté, du dispositif de mesure 1.

La jauge de contrainte 55 est disposée sur la face interne de l'élément souple 53 afin que cette dernière se trouve à l'intérieur de l'anneau ouvert 50 pour éviter tout risque de blessure du vagin.

Le boîtier contient un amplificateur, un convertisseur A/D, un microprocesseur, et une carte mémoire amovible.

Les branches 51, 52 de l'anneau ouvert 50 comportent chacune une extrémité libre sur laquelle une pression peut être exercée pour obtenir une légère déformation de l'élément souple 53 afin de pouvoir placer l'anneau 50 à l'intérieur du vagin de la patiente.

L'anneau ouvert 50 permet du fait de sa structure et de sa composition une adaptation aux différents volumes et circonférences du vagin sans en gêner la circulation sanguine à l'intérieur de ce dernier.

L'anneau ouvert 50 comporte également des moyens d'électro stimulation qui sont constitués par des électrodes 56 positionnées sur la face externe des branches 51, 52.

Les électrodes 56 sont réalisées dans un matériau conducteur qui peut être soit un alliage ou élastomère conducteur. Les électrodes 56 peuvent présenter un profil circulaire ou sphérique ou toute autre forme permettant une bonne électro-stimulation et une bonne mesure de la pression. En effet, les électrodes 56 peuvent servir aussi de capteur de pression.

Les électrodes 56 sont connectées au boîtier externe non représenté comprenant un système d'électro-stimulation et permettant d'envoyer de faibles impulsions électriques aux dits électrodes 56 afin d'assurer une électro-stimulation contrôlée des muscles péri vaginaux (PV) ce qui permet une meilleur mesure, tandis que les informations réceptionnées sont envoyées audit boîtier.

### MUSCLES PERI VAGINAUX (PV), ERECTION CLITORIDIENNE ET MESURES PHYSIOLOGIQUES

L'atrophie des muscles péri vaginaux (PV) comprenant les muscles ischio caverneux (IC) favorise l'anorgasmie féminine. L'incontinence urinaire d'effort est également reliée à l'atrophie de la musculature péri vaginale et des sphincters urétraux qui lui sont associés.

Au cours de l'excitation sexuelle (ES), la pression intra caverneuse (PIC) atteint progressivement la pression systolique moyenne soit 100 à 120 mm/hg. Le volume maximum des corps caverneux clitoridiens est atteint lorsque l'élasticité est bloquée par l'albuginée.

L'albuginée est une membrane de faible élasticité qui entoure le corps caverneux clitoridien empêchant sa dilatation, ce qui permet l'élévation de pression.

La phase vasculaire est la première phase de l'érection clitoridienne c'est la tumescence, conséquence de l'augmentation du débit d'entrée (artériel) dans le corps caverneux clitoridien associée à une diminution du débit de sortie (veineux).

La seconde phase dite phase musculaire devient opérante une fois que la première phase est obtenue. La contraction des muscles péri vaginaux (PV) entourant le corps caverneux clitoridien fait monter la pression intra caverneuse (PIC), ce qui constitue l'érection clitoridienne. Cette élévation de la pression intra caverneuse (PIC) n'est possible que grâce à l'arrêt de la dilatation du corps caverneux clitoridien par l'albuginée.

Pour obtenir une érection clitoridienne, ayant donc une pression intra caverneuse (PIC) élevée due à la contraction des muscles péri vaginaux (PV) la phase vasculaire doit précéder la phase musculaire.

En effet, si la phase vasculaire n'est pas achevée la tension s'exerçant sur l'albuginée est faible et en conséquence son élasticité importante. La contraction musculaire est alors peu ou pas efficace.

Si la pression intra caverneuse (PIC) est faible, le module de Young de l'albuginée est faible, entraînant une faible variation de la pression intra caverneuse (PIC), (delta PIC), provoquée par la contraction des muscles péri vaginaux (PV), car la variation de pression (delta PIC) est absorbée par l'élasticité des tissus.

Par contre, à la fin de la phase vasculaire, si la tumescence est complète la tension s'exerçant sur l'albuginée est forte, son élasticité faible, le module de Young est élevé, provoquant une variation de pression (delta PIC) qui est faiblement absorbée par l'élasticité des tissus et donc des variations de la pression intra caverneuse (PIC) importantes.

Les trois causes les plus fréquentes de dysfonctions sexuelles féminines sont :
- Les causes artérielles dues à la diminution du débit d'entrée,
- Les fuites dues à l'absence de diminution du débit de sortie, ces deux causes étant des perturbations des débits artério-veineux (DAV),
- Les causes musculaires dues à une diminution de la force musculaire des muscles péri vaginaux (PV) par lésion des tissus péri vaginaux, en particulier au moment de l'accouchement.

Le dispositif de mesure 1, comprenant soit un capteur constitué d'une sonde 2 soit un capteur formé d'un anneau ouvert 50, permet de répondre aux besoins énoncés ci-dessus.

### Les causes artérielles ou mesure du débit d'entrée

La mesure du débit d'entrée se fait classiquement à partir de l'effet Doppler. L'effet Doppler mesure la vitesse du sang mais ne mesure pas le débit, à moins de connaître le diamètre des vaisseaux, ce qui est possible mais peu précis. Il est possible de mesurer des variations de débit en mesurant la surface sous la courbe du signal Doppler, mais ce ne sont que des variations.

De plus, les mesures par effet Doppler ne sont pas adaptées aux mesures en continu car la présence de l'investigateur s'impose en permanence. Ceci est dû au fait que la position de la sonde Doppler doit être maintenue avec un angle précis par rapport à l'artère, le moindre mouvement faisant « perdre » le signal.

Le Doppler est donc mal adapté aux mesures en continu surtout au cours du sommeil.

C'est la raison pour laquelle le dispositif de mesure 1 suivant la présente invention permet d'assurer des mesures en continu notamment au cours du sommeil du débit d'entrée par la mesure du volume du pouls (PV) par photo plethysmographie (PVP), mais aussi par mesure des variations de la pression pouls (PP) par tonométrie.

Le dispositif de mesure 1 permet de mesurer les débits artério-veineux (DAV), à savoir le débit des artères caverneuses ou débit d'entrée. Cette méthode est basée sur la mesure de la pression du pouls (PP). En effet, nous observons au cours des enregistrements nocturnes ou diurnes, après absorption d'un vasodilatateur, que les variations de pression engendrées par le pouls sont visibles. Ces variations de pression sont corrélées avec le débit sanguin dans certaines conditions.

Le dispositif de mesure 1 permet de mesurer les débits artério-veineux (DAV) à savoir le débit d'entrée en continu selon deux méthodes différentes :
- soit par volume du pouls (VP) ce qui demande deux capteurs différents, l'un de pression pour mesurer la pression intra caverneuse (PIC) et intra-vaginale et l'autre de PhotoPlethysmographie (PPL) pour mesurer le débit, la PPL étant soit dans la sonde soit déportée à l'extérieur.
- soit par pression du pouls (PP) avec un seul capteur qui mesure simultanément la pression intra caverneuse (PIC) et intra vaginale et les variations de pression de la pression du pouls (PP).

### Les causes de fuites dues à l'absence de diminution du débit de sortie

Les fuites veineuses, ou fuites caverneuses, ou fuites caverno-veineuses sont les principales causes de la diminution du débit de sortie.

Ceci est dû à une diminution de l'élasticité ou compliance du corps caverneux clitoridien qui gène sa dilatation (donc l'érection clitoridienne), mais surtout entrave la diminution des débits artério-veineux (DAV) et plus particulièrement du débit de sortie.

En effet, le réseau veineux du corps caverneux clitoridien qui permet le débit de sortie, est à la périphérie du corps caverneux clitoridien sous l'albuginée, il est donc situé entre le corps caverneux clitoridien et l'albuginée.

Lors de la dilatation du corps caverneux clitoridien dont la compliance est forte le réseau veineux se trouve comprimé entre le corps caverneux clitoridien et l'albuginée dont la compliance est faible.

Si pour des raisons pathologiques, la compliance du corps caverneux clitoridien diminue, la compression veineuse ne peut se faire. Les débits artério-veineux (DAV), et plus particulièrement le débit de sortie ne sont pas réduits, on parle alors de fuite veineuse qui est en fait une fuite caverno-veineuse non pas liée à une pathologie veineuse mais bien à une pathologie caverneuse.

Dans ce cas, même si le débit d'entrée est suffisant, la pression intra caverneuse (PIC) ne peut monter si la fuite est importante. En cas de fuite faible, le débit d'entrée peut compenser cette perte par une augmentation de débit.

Le dispositif de mesure 1 permet de mesurer la fuite à partir de la courbe de la pression intra caverneuse (PIC).

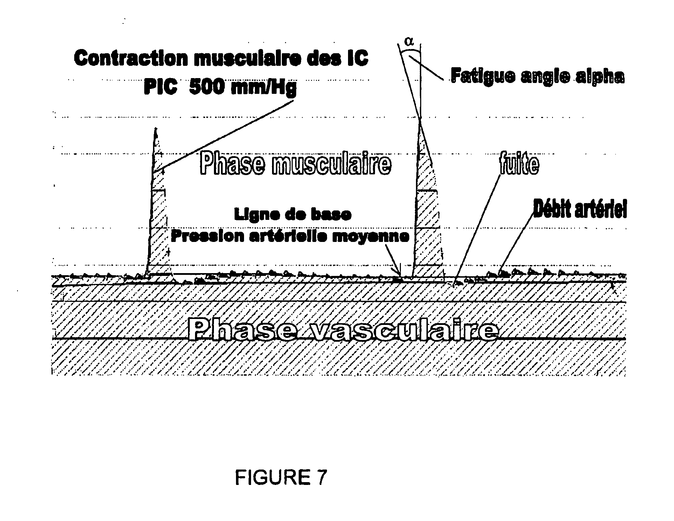

La courbe illustrée en figure 7 représente la pression intra caverneuse (PIC), dont la ligne de base indique le niveau de la pression intra caverneuse (PIC) en dehors d'une contraction des muscles péri vaginaux (PV). L'élévation brutale de la pression correspond à une contraction des muscles péri vaginaux (PV) (contraction musculaire) elle est parfaitement corrélée avec l'activité électromyographique des muscles péri vaginaux (PV).

Au cours du sommeil, ces contractions sont involontaires, au cours d'une séance de rééducation elles sont volontaires, mais l'allure de la courbe est identique.

Le dispositif de mesure 1 permet de mettre en évidence, une chute de la pression intra caverneuse (PIC) en dessous de la ligne de base juste après une contraction volontaire (contraction musculaire), puis une remontée en quelques secondes pour retrouver le niveau de pression obtenu juste avant la contraction.

En effet, lorsque la pression intra caverneuse (PIC) est stable la valeur de la pression intra caverneuse (PIC) n'est autre que la pression systolique moyenne, les débits artério-veineux (DAV), et plus particulièrement le débit d'entrée sont alors égaux aux débits de sortie puisque la pression intra caverneuse (PIC) est stable permettant de mesurer le débit de sortie.

La chute brutale de la pression intra caverneuse (PIC) est provoquée par la contraction musculaire. En effet, en dehors de ces contractions, on n'observe jamais de chute brutale de la pression intra caverneuse (PIC). Cette chute de pression est due à une légère diminution du volume de sang contenu dans le corps caverneux clitoridien.

Il s'agit donc d'une fuite provoquée par la contraction musculaire qui chasse un volume de sang quantifiable. En effet, nous pouvons compter le nombre de battements cardiaques nécessaires à son comblement, connaissant le volume du pouls nous pouvons en déduire le volume de la fuite lors d'une contraction.

Pour une patiente normale, la compliance du corps caverneux (CC) est élevée et la pression intra caverneuse (PIC) comprime le réseau veineux, suffisamment pour permettre aux débits artério-veineux (DAV), et plus particulièrement aux débits de sortie, de rester égaux aux débits d'entrée, c'est la fuite physiologique.

Au cours d'une contraction des muscles péri vaginaux (PV) la brusque élévation de la pression intra caverneuse (PIC) est due à une compression extrinsèque de l'albuginée par les muscles péri vaginaux (PV). Cette pression se transmet bien sur la face interne de l'albuginée puis aux corps caverneux clitoridien, ce qui provoque une élévation brutale de la pression intra caverneuse (PIC) et une forte compression du réseau veineux.

Par contre, si la compliance est faible, la transmission des pressions n'est pas suffisante pour obtenir une compression complète des veines, ce qui chasse un volume de sang en dehors du corps caverneux clitoridien.

La mesure de la fuite se fait à partir de la surface du triangle observée juste après une contraction musculaire volontaire.

Pour une patiente pathologique, surtout lorsqu'on suspecte une fuite, la mesure du module de Young du corps caverneux clitoridien devient fondamentale car elle permet d'évaluer la compliance du corps caverneux clitoridien et donc de confirmer la cause de la fuite.

Il existe plusieurs techniques pour effectuer la mesure du module de Young du corps caverneux clitoridien. Bien évidemment, cette mesure, ainsi que l'évaluation de la compliance du corps caverneux clitoridien peuvent être réalisées avec le dispositif de mesure 1 suivant la présente invention.

### Les causes musculaires dues à une diminution de la force musculaire des muscles péri vaginaux (PV)

La contraction musculaire est bien visible sur la courbe illustrée en figure 7, il s'agit bien d'une contraction musculaire parfaitement corrélée avec l'activité électromyographique des péri vaginaux (PV).

Il existe même un lien de causalité entre les pics enregistrés et la contraction des muscles péri vaginaux (PV). La contraction des muscles péri vaginaux (PV) participe à l'érection clitoridienne

Le dispositif de mesure et de contrôle 1 suivant la présente invention permet, soit la nuit soit au cours d'une érection artificielle, de réaliser les mesures suivantes :
- **Le Pmax** : contraction maximum,
- **Le delta P** : le gradient de pression enregistré au-dessus de la ligne de base,
- **Le delta Moyen** au cours d'une séance ou d'une nuit,
- **La surface** sous la courbe de chaque contraction objectivant le travail fourni par le muscle et la moyenne de ses surfaces,
- **Le nombre de pics,**
- **La largeur de chaque pic** mesurée à sa base et leur moyenne,
- **La mesure de la fuite,**
- **Les débits d'entrée et de sortie,**
- **Les débits artério-veineux (DAV).**

### PROCÉDURE DE L'ENREGISTREMENT NOCTURNE

Le dispositif de mesure 1 constitué du capteur 2, 50 suivant la présente invention permet de suivre l'évolution en mesurant les effets de la rééducation des patientes comportant des dysfonctions orgasmiques ou des incontinences urinaires.

La mesure des variations du tonus péri vaginal du débit artériel péri vaginal des érections clitoridiennes nocturnes est essentielle pour déterminer le type de dysfonction sexuelle à savoir les dysfonctions sexuelles psychogène versus les dysfonctions sexuelles organiques.

Le dispositif de mesure 1 utilisé pour les mesures nocturnes est pratiquement le même que celui utilisé pour la mesure et la surveillance de la rééducation périnéale de jour, il diffère du fait qu'il n'est pas relié à un écran d'ordinateur, que les données sont stockées sur une mémoire amovible, et qu'il n'y a pas de système d'électro-stimulation.

Le logiciel d'analyse est aussi un peu différent, car il calcule notamment la durée des élévations du tonus péri vaginal du débit artériel péri vaginal et des érections clitoridiennes, leur nombre est établi et des zooms pratiqués sur chaque érection.

L'enregistrement se fait à domicile, sans enregistrement de sommeil, mais peut aussi avoir lieu en laboratoire de sommeil.

Si l'enregistrement se fait à domicile, le boîtier est confié à la patiente qui vient le chercher chez le médecin.

Au préalable, le médecin aura programmé le boîtier afin d'enregistrer les caractéristiques de la patiente (date, nom, prénom, age, tabac, les pathologies sont détaillées, glycémie cholestérol médications, et toutes autres informations susceptibles d'intervenir sur sa fonction sexuelle).

Egalement, le médecin pourra isoler à l'aide du centre d'analyse et de télé contrôle (CAT) des groupes de patientes ayant un même type de pathologie dit « Clusters » musculaires, vasculaires, mixtes, normaux, dont on établira les moyennes et écarts types, chaque nouvel enregistrement venant s'ajouter automatiquement à son cluster, et réactualiser sa statistique.

Le médecin, après avoir enregistré les données de la patiente, vérifie la charge des piles, le calibrage, et le bon fonctionnement de l'enregistrement. Il déconnecte le boîtier de son ordinateur et le confie à la patiente.

Au coucher, la patiente place la sonde 2 dans son vagin qui est connecté par une liaison sans fil ou avec fil au boîtier, une LED rouge clignote indiquant que l'enregistrement a commencé. Un zéro automatique du capteur de pression 8 est réalisé par le logiciel après quelques minutes d'enregistrement.

Au réveil, la patiente vérifie que la LED est toujours allumée attestant le fait que l'enregistrement n'ait pas été interrompu au cours de la nuit. Elle met le boîtier sur arrêt.

La patiente recommence les mêmes opérations décrites ci-dessus les nuits suivantes.

Après trois nuits consécutives, la patiente ramène le boîtier chez le médecin qui bascule les données contenues dans la mémoire du boîtier sur son ordinateur.

Les données sont analysées partiellement par le logiciel puis envoyées, via Internet, dans la base de données du centre d'analyse et de télé contrôle (CAT).

Une sortie papier des courbes de chaque nuit, des zooms et des calculs sera imprimée, l'analyse complète sera envoyée par le centre d'analyse et de télé contrôle (CAT).

### PROCÉDURE POUR LA MESURE ET/OU LA SURVEILLANCE DE LA REEDUCATION DES DYSFONCTIONS SEXUELLES

Le dispositif de mesure 1 constitué du capteur 2, 50 suivant la présente invention permet une mesure et/ou une surveillance des effets de la rééducation des patientes comportant des dysfonctions sexuelles et incontinences. Cette mesure et/ou la surveillance des effets de la rééducation peut être réalisées soit au cabinet du médecin traitant soit au domicile de la patiente.

Lorsque l'analyse des dysfonctions sexuelles organiques est établie par le dispositif de mesure 1, ce dernier permet aussi la surveillance des effets de la rééducation.

La mesure et/ou la surveillance des effets de la rééducation sont indiquées spécialement pour les patientes présentant une pathologie musculaire ayant une phase vasculaire normale, mais aussi pour celles ayant une pathologie mixte vasculaire d'origine artérielle et musculaire.

La patiente est placée sur un lit d'examen ou sur son lit, la sonde 2 introduite dans son vagin de sorte que les lamelles d'électro-stimulation 7 et le capteur de pression 8 soient bien en regard des muscles péri vaginaux, ce qui est réalisé grâce à la collerette 5 qui vient en appui sur la vulve permettant son bon positionnement.

Dans le cas de l'utilisation d'un anneau ouvert 50 la patiente est placée sur un lit d'examen ou sur son lit, ledit anneau ouvert est introduit dans son vagin de sorte que les branches 51, 52 pourvues des électrodes d'électro stimulation 56 soient bien en regard des muscles péri vaginaux (PV).

Le boîtier est relié par un câble USB ou par un système bluetooth ou par un autre système à un ordinateur. Le dispositif est allumé et le médecin procède à la mise à zéro du capteur de pression 8, soit automatiquement après une minute de fonctionnement, soit à l'aide d'un bouton virtuel situé sur l'écran soit de l'ordinateur, soit d'un PDA, soit d'un téléphone portable permettant de piloter le boîtier (arrêt, marche, zéro, électro-stimulation).

Le système d'électro-stimulation de la carte électronique 15 est mis en route, et la patiente règle l'intensité de la stimulation soit à l'aide d'un potentiomètre placé sur le boîtier soit à l'aide d'un bouton virtuel situé sur l'écran soit de l'ordinateur, soit d'un PDA, soit d'un téléphone portable.

L'électrostimulation se poursuit jusqu'à la fin de la séance, car elle améliore les performances de la mesure et de la surveillance des effets de la rééducation des muscles péri vaginaux (PV) ainsi que la vasodilatation péri vaginale.

La patiente doit alors vérifier que ses contractions volontaires provoquent bien une élévation de la pression sur le capteur de pression 8 de la sonde 2 ce qui prouve que les bons muscles sont contractés car seuls les muscles péri vaginaux (PV) doivent être contractés, sans contraction des muscles abdominaux qui perturbent les mesures.

La patiente voit alors apparaître à l'écran un masque de contractions musculaires qu'elle est invitée à suivre, il lui indique la durée et la hauteur optimale de la contraction qui doit être faite.

Ce masque modulable est établi à partir de la courbe de fatigue des muscles péri vaginaux (PV). Il permet d'optimiser la rééducation. En effet, un travail insuffisant sera synonyme d'absence d'amélioration mais à l'inverse une rééducation trop intense va épuiser les muscles péri vaginaux (PV). Il existe donc une intensité optimale calculée en fonction de la courbe de fatigue.

La fatigue des muscles péri vaginaux (PV) se calcule en mesurant l'angle α matérialisé sur la courbe. Pour une largeur de pic donnée, plus l'angle est petit plus la fatigue est importante. Les modifications du gabarit indiquant les modalités de la contraction à effectuer seront calculées soit en temps réel au cours de la séance soit entre deux séances lorsque cette analyse sera faite par le centre d'analyse et de télé contrôle (CAT).

Toute cette procédure permet de mesurer et de surveiller les effets de la rééducation des muscles péri vaginaux (PV).

### PROCÉDURE POUR LA MESURE ET/OU LA SURVEILLANCE DE LA REEDUCATION DES PROBLEMES D'INCONTINENCE URINAIRE D'EFFORT

La mesure et/ou la surveillance des effets de la rééducation périnéale est obtenue au moyen du dispositif de mesure 1 avec électro-stimulation du vagin par l'intermédiaire des moyens d'électro-stimulation 7, 56 positionnées sur le capteur 2, 50. Cette électro-stimulation du vagin permet de renforcer les muscles péri vaginaux (PV) entourant le vagin, de pratiquer des mesures dans les conditions optimales et de favoriser l'augmentation de la circulation artérielle.

La mesure et/ou la surveillance des effets de la rééducation peut par exemple être réalisée au domicile de la patiente qui possède une sonde 2 pourvue de sa carte électronique 15 ou d'un anneau ouvert 50 connectée au boîtier. Ce dernier peut être connecté via bluetooth à son ordinateur ou à son PDA ou à son téléphone portable, comme pour le traitement des dysérections.

La séance étant conçue de la même manière que précédemment.

La patiente est invitée à placer la sonde 2 ou l'anneau ouvert 50 à l'intérieur de son vagin de manière que les électrodes d'électro stimulation 7, 56 et le capteur de pression soient en contact avec les muscles péri vaginaux (PV). La patiente met le boîtier en marche, s'assure que la connexion fonctionne en vérifiant qu'une variation de pression se traduit bien (en appuyant sur la sonde) par une élévation de pression sur l'écran.

Il doit d'ailleurs être entendu que la description qui précède n'a été donnée qu'a titre d'exemple et qu'elle ne limite nullement le domaine de l'invention dont on ne sortirait pas en remplaçant les détails d'exécution décrits par tous autres équivalents.

## Revendications

1. Procédé de mesure permettant de mesurer les signaux physiologiques vaginaux et péri vaginaux comme les pressions et les variations de pressions lors d'une contraction des muscles péri vaginaux (PV), mais aussi le pouls péri vaginal et clitoridien, la pression intra caverneuse clitoridienne, la température vaginale mais aussi tout autre signal physiologique pertinent au moyen d'un dispositif de mesure suivant l'une quelconque des revendications 11 à 21; le procédé comprenant les étapes suivantes
• Placer un capteur (2, 50) comportant des moyens pour capter la pression et les variations de pressions lors d'une contraction des muscles péri vaginaux (PV), et au moins un transducteur (11, 13, 55) appartenant au capteur permettant de transformer les signaux de pression issus des moyens pour capter la pression en signaux électriques,
• Et à connecter le capteur (2, 50) à une carte électronique (15) ou à un boîtier comportant des moyens d'amplification et d'enregistrement des signaux provenant des transducteurs appartenant au capteur, des moyens d'analyse des signaux par un microcontrôleur permettant de mesurer l'état de fatigue des muscles péri vaginaux (PV), de mesurer les variations de la force musculaire des muscles péri vaginaux (PV) de mesurer le module de Young du corps caverneux clitoridien permettant d'évaluer la compliance dudit corps caverneux clitoridien.

2. Procédé de mesure suivant la revendication 1, **caractérisé en ce qu'**il consiste à mesurer les variations de la pression intra caverneuse (PIC) afin de quantifier les variations du tonus des muscles péri vaginaux et de leur circulation artérielle et veineuse de jour comme de nuit.

3. Procédé de mesure suivant la revendication 1, **caractérisé en ce qu'**il consiste à mesurer la pression intra caverneuse (PIC) lorsqu'elle atteint progressivement la pression systolique moyenne.

4. Procédé de mesure suivant la revendication 1, **caractérisé en ce qu'**il consiste à mesurer pendant la phase vasculaire qui est la première phase de l'érection clitoridienne, l'augmentation du débit artériel d'entrée dans le corps caverneux clitoridien et la diminution du débit veineux de sortie.

5. Procédé de mesure suivant la revendication 1, **caractérisé en ce qu'**il consiste à mesurer pendant la phase musculaire qui est la seconde phase de l'érection clitoridienne, les variations de la contraction des muscles péri vaginaux (PV), dont les muscles ischio caverneux (IC), entourant le corps caverneux clitoridien.

6. Procédé de mesure suivant la revendication 1, **caractérisé en ce qu'**il consiste à mesurer en continu le débit artériel d'entrée et le débit veineux de sortie par la mesure de la pression du pouls (PP) et du volume du pouls (VP).

7. Procédé de mesure suivant la revendication 1, **caractérisé en ce qu'**il consiste à mesurer la fuite cavemo veineuse à partir de la courbe de la pression intra caverneuse (PIC).

8. Procédé de mesure suivant la revendication 1, **caractérisé en ce qu'**il consiste à mesurer la contraction maximum (Pmax), le gradient de pression (Delta P et Delta Moyen), la surface sous la courbe traduisant le travail musculaire de chaque contraction des muscles péri vaginaux, le nombre de pics et la largeur de chaque pic.

9. Procédé de mesure suivant la revendication 1, **caractérisé en ce qu'**il consiste à mesurer la fatigue des muscles péri vaginaux (PV) en mesurant l'angle (*α*).

10. Procédé de mesure suivant la revendication 1, **caractérisé en ce qu'**il consiste à mesurer les pics des contractions des muscles vaginaux (PV) et les variations de pression du pouls (PP) et de volume du pouls (VP).

11. Dispositif de mesure pour la mise en oeuvre du procédé suivant l'une quelconque des revendications 1 à 10, le dispositif comprenant un capteur constitué d'une sonde (2) comportant un corps longitudinal cylindrique (4) pourvu d'une collerette (5) formant butée, un capteur de pression (8) placé suivant une direction longitudinale à la surface du corps cylindrique (4), ledit capteur de pression (8) comprenant au moins un transducteur permettant de transformer les signaux de pression issus des moyens pour capter la pression en signaux électriques, une carte électronique (15) disposée à l'intérieur du corps cylindrique (4) et à laquelle est connecté le capteur de pression (8), et des moyens de communication traversant longitudinalement la sonde (2) qui sont constitués par au moins une cannelure longitudinale (16) ménagée sur la périphérie du corps cylindrique (4) et traversant la collerette (5) pour permettre de relier la partie interne du vagin à l'extérieur afin d'équilibrer les pressions intra vaginales et extra vaginales, ladite carte électronique (15) comportant des moyens d'amplification et d'enregistrement des signaux provenant des transducteurs, des moyens d'analyse des signaux par un microcontrôleur permettant de mesurer l'état de fatigue des muscles péri vaginaux (PV), de mesurer les variations de la force musculaire des muscles péri vaginaux (PV) et de mesurer le module de Young du corps caverneux clitoridien permettant d'évaluer la compliance dudit corps caverneux clitoridien.

12. Dispositif de mesure pour la mise en oeuvre du procédé suivant l'une quelconque des revendications 1 à 10, le dispositif comprenant un capteur constitué d'une sonde (2) comportant un corps longitudinal cylindrique (4) pourvu d'une collerette (5) formant butée, un capteur de pression (8) placé suivant une direction longitudinale à la surface du corps cylindrique (4), ledit capteur de pression (8) comprenant au moins un transducteur permettant de transformer les signaux de pression issus des moyens pour capter la pression en signaux électriques, une carte électronique (15) disposée à l'intérieur du corps cylindrique (4) et à laquelle est connecté le capteur de pression (8), et des moyens de communication traversant longitudinalement la sonde (2) qui sont constitués par un canal (17) ménagé à l'intérieur du corps cylindrique (4) et traversant longitudinalement ce dernier et la collerette (5) pour permettre de relier la partie interne du vagin à l'extérieur afin d'équilibrer les pressions intra vaginales et extra vaginales, ladite carte électronique (15) comportant des moyens d'amplification et d'enregistrement des signaux provenant des transducteurs, des moyens d'analyse des signaux par un microcontrôleur permettant de mesurer l'état de fatigue des muscles péri vaginaux (PV), de mesurer les variations de la force musculaire des muscles péri vaginaux (PV) et de mesurer le module de Young du corps caverneux clitoridien permettant d'évaluer la compliance dudit corps caverneux clitoridien.

13. Dispositif de mesure pour la mise en oeuvre du procédé suivant l'une quelconque des revendications 1 à 10, le dispositif comprenant un capteur constitué d'une sonde (2) comportant un corps longitudinal cylindrique (4) pourvu d'une collerette (5) formant butée, un capteur de pression (8) placé suivant une direction longitudinale à la surface du corps cylindrique (4) ledit capteur de pression (8) comprenant au moins un transducteur permettant de transformer les signaux de pression issus des moyens pour capter la pression en signaux électriques, une carte électronique (15) disposée à l'intérieur du corps cylindrique (4) et à laquelle est connecté le capteur de pression (8), et des moyens de communication traversant longitudinalement la sonde (2) qui sont constitués d'une part par au moins une cannelure longitudinale (16) ménagée sur la périphérie du corps cylindrique (4) et traversant la collerette (5) et d'autre part par un canal (17) ménagé à l'intérieur du corps cylindrique (4) et traversant longitudinalement ce dernier et la collerette (5), lesdits moyens de communication permettant de relier la partie interne du vagin à l'extérieur afin d'équilibrer les pressions intra vaginales et extra vaginales, ladite carte électronique (15) comportant des moyens d'amplification et d'enregistrement des signaux provenant des transducteurs, des moyens d'analyse des signaux par un microcontrôleur permettant de mesurer l'état de fatigue des muscles péri vaginaux (PV), de mesurer les variations de la force musculaire des muscles péri vaginaux (PV) et de mesurer le module de Young du corps caverneux clitoridien permettant d'évaluer la compliance dudit corps caverneux clitoridien.

14. Dispositif de mesure suivant l'une quelconque des revendications 11 à 13, **caractérisé en ce que** la sonde (2) comporte à la surface du corps cylindrique (4) des lamelles d'électro-stimulation (7) connectées à la carte électronique (15).

15. Dispositif de mesure suivant la revendication 14, **caractérisé en ce que** les lamelles d'électro-stimulation (7) sont positionnées parallèlement à l'axe longitudinal dudit corps cylindrique (4).

16. Dispositif de mesure suivant la revendication 14, **caractérisé en ce que** les lamelles d'électro-stimulation (7) présentent un profil circulaire ou sphérique.

17. Dispositif de mesure suivant l'une quelconque des revendications 11 à 13, **caractérisé en ce que** le capteur de pression (8) est constitué d'une lamelle métallique (9) qui est fixée dans une rainure (10) ménagée dans le corps cylindrique (4), ladite rainure (10) étant remplie d'un matériau composite élastique (12) permettant d'absorber les mouvements ou les déformations élastiques de ladite lamelle (9), tandis que la face interne de la lamelle métallique (9) est solidaire d'une jauge de contrainte (11) ou transducteur permettant de transformer le signal de pression issu de la lamelle métallique (9) en un signal électrique.

18. Dispositif de mesure suivant l'une quelconque des revendications 11 à 13, **caractérisé en ce que** le capteur de pression (8) est constitué de transducteurs formant un film piezzo électrique (13) fixé au niveau de la rainure (10) remplie d'un matériau composite élastique (12).

19. Dispositif de mesure suivant l'une quelconque des revendications 11 à 13, **caractérisé en ce que** la sonde (2) comporte à l'intérieur du corps cylindrique (4) un évidemment (14) permettant la mise en place et la retenue d'une carte électronique (15).

20. Dispositif de mesure suivant l'une quelconque des revendications 11 à 13, **caractérisé en ce que** la sonde (2) comporte tous les capteurs nécessaires aux mesures physiologiques vaginales connectés à la carte électronique (15).

21. Dispositif de mesure suivant l'une quelconque des revendications 11 à 13, **caractérisé en ce que** la carte électronique (15) peut être raccordée à un boîtier contenant un ou des ampliflcateur(s), un convertisseur A/D, et un microprocesseur.

## Claims

1. A measuring method making it possible to measure vaginal and perivaginal physiological signals such as the pressures and pressure variations during a contraction of the perivaginal (PV) muscles, as well as the perivaginal and clitoral pulse, the clitoral intra-cavernous pressure, the vaginal temperature, as well as any other relevant physiological signal using a measuring device according to any one of claims 11 to 21, the method comprising the following steps:
• Placing a sensor (2, 50) including means for sensing the pressure and the pressure variations during a contraction of the perivaginal (PV) muscles, and at least one transducer (11, 13, 55) belonging to the sensor making it possible to convert the pressure signals from the pressure sensing means into electrical signals,
• And connecting the sensor (2, 50) to an electronic board (15) or to a housing including means for amplifying and recording signals coming from the transducers belonging to the sensor, means for analyzing signals using a microcontroller making it possible to measure the fatigue state of the perivaginal (PV) muscles, measuring the variations of the muscular force of the perivaginal (PV) muscles and measuring the Young's modulus of the clitoral cavernous body making it possible to evaluate the compliance of said clitoral cavernous body.

2. The measuring method according to claim 1, **characterized in that** it consists of measuring the intra-cavernous pressure (ICP) variations so as to quantify the variations of the tonus of the perivaginal muscles and their arterial and venous circulation day and night.

3. The measuring method according to claim 1, **characterized in that** it consists of measuring the intra-cavernous pressure (ICP) when it gradually reaches the mean systolic pressure.

4. The measuring method according to claim 1, **characterized in that** it consists of measuring, during the vascular phase, which is the first phase of clitoral erection, the increase in the arterial flow rate entering the clitoral cavernous body and the decrease of the venous flow rate leaving it.

5. The measuring method according to claim 1, **characterized in that** it consists of measuring, during the muscular phase, which is the second phase of clitoral erection, the variations of the contraction of the perivaginal muscles (PV), including the ischiocavernous (IC) muscles, surrounding the clitoral cavernous body.

6. The measuring method according to claim 1, **characterized in that** it consists of continuously measuring the entering arterial flow rate and the outgoing venous flow rate by measuring the pulse pressure (PP) and the volume of the pulse (VP).

7. The measuring method according to claim 1, **characterized in that** it consists of measuring the cavernous-venous leakage from the curve of the intra-cavernous pressure (ICP).

8. The measuring method according to claim 1, **characterized in that** it consist of measuring the maximum contraction (Pmax), the pressure gradient (Delta P and Delta Mean), the surface under the curve translating the muscular work of each contraction of the perivaginal muscles, the number of peaks, and the width of each peak.

9. The method according to claim 1, **characterized in that** it consists of measuring the fatigue of the perivaginal (PV) muscles by measuring the angle (α).

10. The measuring method according to claim 1, **characterized in that** it consists of measuring the peaks of the contractions of the perivaginal (PV) muscles and the variations in the pulse pressure (PP) and the volume of the pulse (VP).

11. A device for measuring the implementation of the method according to any one of claims 1 to 10, the device comprising a sensor made up of a probe (2) having a cylindrical longitudinal body (4) provided with a flange (5) forming a stop, a pressure sensor (8) placed along a longitudinal direction at the surface of the cylindrical body (4), said pressure sensor (8) comprising at least one transducer making it possible to convert the pressure signals coming from the means for sensing the pressure into electrical signals, an electronic board (15) arranged inside the cylindrical body (4) and to which the pressure sensor (8) is connected, and communication means passing longitudinally through the probe (2) which are made up of at least one longitudinal rib (16) formed on the periphery of the cylindrical body (4) and passing through the flange (5) to make it possible to connect the inner portion of the vagina to the outside so as to balance the intravaginal and extravaginal pressures, said electronic board (15) including means for amplifying and recording signals coming from the transducers, means for analyzing signals using a microcontroller making it possible to measure the fatigue state of the perivaginal (PV) muscles, measure the variations in the muscular strength of the perivaginal (PV) muscles, and measure the Young's modulus of the clitoral cavernous body making it possible to evaluate the compliance of said clitoral cavernous body.

12. A measuring device for implementing the method according to any one of claims 1 to 10, the device comprising a sensor made up of a probe (2) including a cylindrical longitudinal body (4) provided with a flange (5) forming a stop, a pressure sensor (8) placed along a longitudinal direction at the surface of the cylindrical body (4), said pressure sensor (8) comprising at least one transducer making it possible to convert the pressure signals from the means for sensing the pressure into electrical signals, an electronic board (15) arranged inside the cylindrical body (4) and to which the pressure sensor (8) is connected, and communication means passing longitudinally through the probe (2) which are made up of a channel (17) formed inside the cylindrical body (4) and longitudinally passing through the latter and the flange (5) to make it possible to connect the inner portion of the vagina to the outside so as to balance the intravaginal and extravaginal pressures, said electronic board (15) including means for amplifying and recording signals coming from the transducers, means for analyzing signals using a microcontroller making it possible to measure the fatigue state of the perivaginal (PV) muscles, measure the variations in the muscular strength of the perivaginal (PV) muscles, and measure the Young's modulus of the clitoral cavernous body making it possible to evaluate the compliance of said clitoral cavernous body.

13. A measuring device for implementing the method according to any one of claims 1 to 10, the device comprising a sensor made up of a probe (2) including a cylindrical longitudinal body (4) provided with a flange (5) forming a stop, a pressure sensor (8) placed along a longitudinal direction at the surface of the cylindrical body (4), said pressure sensor (8) comprising at least one transducer making it possible to convert the pressure signals from the means for sensing the pressure into electrical signals, an electronic board (15) arranged inside the cylindrical body (4) and to which the pressure sensor (8) is connected, and communication means passing longitudinally through the probe (2) which are made up on the one hand by at least one longitudinal rib (16) formed on the periphery of the cylindrical body (4) and passing through the flange (5) and on the other hand by a channel (17) formed inside the cylindrical body (4) and longitudinally passing through the latter and the flange (5), said communication means making it possible to connect the inner portion of the vagina to the outside so as to balance the intravaginal and extravaginal pressures, said electronic board (15) including means for amplifying and recording signals coming from the transducers, means for analyzing signals using a microcontroller making it possible to measure the fatigue state of the perivaginal (PV) muscles, measure the variations in the muscular strength of the perivaginal (PV) muscles, and measure the Young's modulus of the clitoral cavernous body making it possible to evaluate the compliance of said clitoral cavernous body.

14. The measuring device according to any one of claims 11 to 13, **characterized in that** the probe (2) includes, on the surface of the cylindrical body (4), electrostimulation strips (7) connected to the electronic board (15).

15. The measuring device according to claim 14, **characterized in that** the electrostimulation strips (7) are positioned parallel to the longitudinal axis of said cylindrical body (4).

16. The measuring device according to claim 14, **characterized in that** the electrostimulation strips (7) have a circular or spherical profile.

17. The measuring device according to any one of claims 11 to 13, **characterized in that** the pressure sensor (8) is made up of a metal strip (9) that is fastened on a slot (10) formed in the cylindrical body (4), said slot (10) being filled with an elastic composite material (12) making it possible to absorb the movements or elastic deformations of said strip (9), while the inner surface of the metal strip (9) is secured to a strain gauge (11) or transducer making it possible to convert the pressure signal from the metal strip (9) into an electrical signal.

18. The measuring device according to any one of claims 11 to 13, **characterized in that** the pressure sensor (8) is made up of transducers forming a piezoelectric film (13) fastened at the slot (10) filled with an elastic composite material (12).

19. The measuring device according to any one of claims 11 to 13, **characterized in that** the probe (2) includes, include the cylindrical body (4), a recess (14) making it possible to place and retain an electronic board (15).

20. The measuring device according to any one of claims 11 to 13, **characterized in that** the probe (2) includes all of the sensors necessary for the physiological vaginal measurements connected to the electronic board (15).

21. The measuring device according to any one of claims 11 to 13, **characterized in that** the electronic board (15) can be connected to a housing containing one or more amplifiers, an A/D converter, and a microprocessor.

## Patentansprüche

1. Messverfahren, das es erlaubt, die vaginalen und perivaginalen physiologischen Signale wie die Drücke und die Druckschwankungen bei einer Kontraktion der perivaginalen Muskeln (PV), aber auch den perivaginalen und Klitorispuls, den intrakavernösen Klitorisdruck, die Vaginaltemperatur, aber auch jedes andere physiologisch relevante Signal mittels einer Messvorrichtung nach einem der Ansprüche 11 bis 21 zu messen, wobei das Verfahren die folgenden Schritte aufweist:
• Platzieren eines Sensors (2, 50), der Mittel aufweist, um den Druck und die Druckschwankungen bei einer Kontraktion der perivaginalen Muskeln aufzunehmen, und mindestens einen Messgrößenumformer (11, 13, 55), der zum Sensor gehört und erlaubt, die Drucksignale, die von den Empfangsmitteln des Drucks ausgegeben werden, in elektrische Signale umzuwandeln,
• und Verbinden des Sensors (2, 50) mit einer elektronischen Karte (15) oder einem Gehäuse, das Verstärkungs- und Aufzeichnungsmittel der von den Messgrößenumformern, die zum Sensor gehören, kommenden Signale aufweist, wobei Mittel für die Analyse der Signale durch einen Mikrocontroller erlauben, den Ermüdungszustand der perivaginalen Muskeln (PV) zu messen, die Schwankungen der Muskelkraft der perivaginalen Muskeln (PV) zu messen und das Young-Modul des kavernösen Klitoriskörpers zu messen, was erlaubt, die Compliance des kavernösen Klitoriskörpers zu bewerten.

2. Messverfahren nach Anspruch 1, **dadurch gekennzeichnet, dass** es darin besteht, die intrakavernösen Druckschwankungen (PIC) zu messen, um die Schwankungen des Tonus der perivaginalen Muskeln und ihrer arteriellen und venösen Zirkulation am Tag wie in der Nacht zu quantifizieren.

3. Messverfahren nach Anspruch 1, **dadurch gekennzeichnet, dass** es darin besteht, den intrakavernösen Druck (PIC) zu messen, wenn er schrittweise den durchschnittlichen systolischen Druck erreicht.

4. Messverfahren nach Anspruch 1, **dadurch gekennzeichnet, dass** es darin besteht, während der vaskulären Phase, die die erste Phase der Klitoriserektion ist, die Erhöhung des arteriellen Eingangsdurchsatzes im kavernösen Klitoriskörper und die Verringerung des venösen Ausgangsdurchsatzes zu messen.

5. Messverfahren nach Anspruch 1, **dadurch gekennzeichnet, dass** es darin besteht, während der muskulären Phase, die die zweite Phase der Klitoriserektion ist, die Kontraktionsschwankungen der perivaginalen Muskeln (PV) zu messen, darunter auch der ischiokavernösen Muskeln (IC), die den kavernösen Klitoriskörper umschließen.

6. Messverfahren nach Anspruch 1, **dadurch gekennzeichnet, dass** es darin besteht, kontinuierlich den arteriellen Eingangsdurchsatz und den venösen Ausgangsdurchsatz durch Messen des Drucks des Pulses (PP) und des Volumens des Pulses (VP) zu messen.

7. Messverfahren nach Anspruch 1, **dadurch gekennzeichnet, dass** es darin besteht, ausgehend von der Kurve des intrakavernösen Drucks (PIC) den kavernovenösen Verlust zu messen.

8. Messverfahren nach Anspruch 1, **dadurch gekennzeichnet, dass** es darin besteht, die maximale Kontraktion (Pmax), den Druckgradienten (Delta P und Delta Mittel) zu messen, wobei die Fläche unter der Kurve die Muskelarbeit jeder Kontraktion der perivaginalen Muskeln, die Anzahl der Scheitelpunkte und die Breite jedes Scheitelpunkts veranschaulicht.

9. Messverfahren nach Anspruch 1, **dadurch gekennzeichnet, dass** es darin besteht, die Ermüdung der perivaginalen Muskeln (PV) durch Messen des Winkels (α) zu messen.

10. Messverfahren nach Anspruch 1, **dadurch gekennzeichnet, dass** es darin besteht, die Scheitelpunkte der Kontraktionen der perivaginalen Muskeln (PV) und die Schwankungen des Drucks des Pulses (PP) und des Volumen des Pulses (VP) zu messen.

11. Messvorrichtung zur Umsetzung des Verfahrens nach einem der Ansprüche 1 bis 10, wobei die Vorrichtung einen Sensor umfasst, der von einer Sonde (2) gebildet wird, die einen zylindrischen länglichen Körper (4) aufweist, der mit einem Ring (5), der einen Anschlag bildet, ausgestattet ist, einen Drucksensor (8), der gemäß einer Längsrichtung auf der Oberfläche des zylindrischen Körpers (4) platziert ist, wobei der Drucksensor (8) mindestens einen Messgrößenumformer umfasst, der es erlaubt, die Drucksignale, die von den Empfangsmitteln des Drucks ausgegeben werden, in elektrische Signale umzuwandeln, eine elektronische Karte (15), die im zylindrischen Körper (4) angeordnet ist und an die der Drucksensor (8) gekoppelt ist und Kommunikationsmittel, die die Sonde (2) längs durchqueren, die von mindestens einer Längsrille (16) gebildet werden, die auf dem Umfang des zylindrischen Körpers (4) ausgebildet ist und den Ring (5) durchquert, um die Verbindung des inneren Teils der Vagina mit der Außenwelt zu erlauben, um die intra- und extravaginalen Drücke auszugleichen, wobei die elektronische Karte (15) Verstärkungs- und Aufzeichnungsmittel der Signale aufweist, die von den Messgrößenumformern geliefert werden, wobei Mittel für die Analyse der Signale durch einen Mikrocontroller erlauben, den Ermüdungszustand der perivaginalen Muskeln (PV) zu messen, die Schwankungen der Muskelkraft der perivaginalen Muskeln (PV) zu messen und das Young-Modul des kavernösen Klitoriskörpers zu messen, was erlaubt, die Compliance des kavernösen Klitoriskörpers zu bewerten.

12. Messvorrichtung zur Umsetzung des Verfahrens nach einem der Ansprüche 1 bis 10, wobei die Vorrichtung einen Sensor umfasst, der von einer Sonde (2) gebildet wird, die einen zylindrischen länglichen Körper (4) aufweist, der mit einem Ring (5), der einen Anschlag bildet, ausgestattet ist, einen Drucksensor (8), der gemäß einer Längsrichtung auf der Oberfläche des zylindrischen Körpers (4) platziert ist, wobei der Drucksensor (8) mindestens einen Messgrößenumformer umfasst, der es erlaubt, die Drucksignale, die von den Empfangsmitteln des Drucks ausgehen, in elektrische Signale umzuwandeln, eine elektronische Karte (15), die im zylindrischen Körper (4) angeordnet ist und an die der Drucksensor (8) gekoppelt ist und Kommunikationsmittel, die die Sonde (2) längs durchqueren, die von einem Kanal (17) gebildet werden, der in dem zylindrischen Körper (4) ausgebildet ist und diesen und den Ring (5) längs durchquert, um die Verbindung des inneren Teils der Vagina mit der Außenwelt zu erlauben, um die intra- und extravaginalen Drücke auszugleichen, wobei die elektronische Karte (15) Verstärkungs- und Aufzeichnungsmittel der Signale aufweist, die von den Messgrößenumformern geliefert werden, wobei Mittel für die Analyse der Signale durch einen Mikrocontroller erlauben, den Ermüdungszustand der perivaginalen Muskeln (PV) zu messen, die Schwankungen der Muskelkraft der perivaginalen Muskeln (PV) zu messen und das Young-Modul des kavernösen Klitoriskörpers zu messen, was erlaubt, die Compliance des kavernösen Klitoriskörpers zu bewerten.

13. Messvorrichtung zur Umsetzung des Verfahrens nach einem der Ansprüche 1 bis 10, wobei die Vorrichtung einen Sensor umfasst, der von einer Sonde (2) gebildet wird, die einen zylindrischen länglichen Körper (4) aufweist, der mit einem Ring (5), der einen Anschlag bildet, ausgestattet ist, einen Drucksensor (8), der gemäß einer Längsrichtung auf der Oberfläche des zylindrischen Körpers (4) platziert ist, wobei der Drucksensor (8) mindestens einen Messgrößenumformer umfasst, der es erlaubt, die Drucksignale, die von den Empfangsmitteln des Drucks ausgehen, in elektrische Signale umzuwandeln, eine elektronische Karte (15), die im zylindrischen Körper (4) angeordnet ist und an die der Drucksensor (8) gekoppelt ist und Kommunikationsmittel, die die Sonde (2) längs durchqueren, die einerseits von mindestens einer länglichen Rille (16) gebildet werden, die auf dem Umfang des zylindrischen Körpers (4) ausgebildet ist und den Ring (5) durchquert und andererseits von einem Kanal (17), der in dem zylindrischen Körper (4) ausgebildet ist und diesen und den Ring (5) längs durchquert, wobei es die Kommunikationsmittel erlauben, den inneren Teil der Vagina mit der Außenwelt zu verbinden, um die intra- und extravaginalen Drücke auszugleichen, wobei die elektronische Karte (15) Verstärkungs- und Aufzeichnungsmittel der Signale aufweist, die von den Messgrößenumformern geliefert werden, wobei Mittel für die Analyse der Signale durch einen Mikrocontroller erlauben, den Ermüdungszustand der perivaginalen Muskeln (PV) zu messen, die Schwankungen der Muskelkraft der perivaginalen Muskeln (PV) zu messen und das Young-Modul des kavernösen Klitoriskörpers zu messen, was erlaubt, die Compliance des kavernösen Klitoriskörpers zu bewerten.

14. Messvorrichtung nach einem der Ansprüche 11 bis 13, **dadurch gekennzeichnet, dass** die Sonde (2) auf der Oberfläche des zylindrischen Körpers (4) Elektrostimulationslamellen (7) aufweist, die an die elektronische Karte (15) gekoppelt sind.

15. Messvorrichtung nach Anspruch 14, **dadurch gekennzeichnet, dass** die Elektrostimulationslamellen (7) parallel zur Längsachse des zylindrischen Körpers (4) positioniert sind.

16. Messvorrichtung nach Anspruch 14, **dadurch gekennzeichnet, dass** die Elektrostimulationslamellen (7) ein kreisförmiges oder kugelförmiges Profil aufweisen.

17. Messvorrichtung nach einem der Ansprüche 11 bis 13, **dadurch gekennzeichnet, dass** der Drucksensor (8) aus einer Metalllamelle (9) besteht, die in einer Rille (10) befestigt ist, die in dem zylindrischen Körper (4) ausgebildet ist, wobei die Rille (10) mit einem elastischen Verbundmaterial (12) gefüllt ist, das es erlaubt, die Bewegungen oder die elastischen Verformungen der Lamelle (9) zu absorbieren, wogegen die Innenseite der Metalllamelle (9) mit einem Spannungsfühler (11) oder Messgrößenumformer verbunden ist, der es erlaubt, das von der Metalllamelle (9) ausgegebene Drucksignal in ein elektrisches Signal umzuwandeln.

18. Messvorrichtung nach einem der Ansprüche 11 bis 13, **dadurch gekennzeichnet, dass** der Drucksensor (8) aus Messgrößenumformern besteht, die eine piezoelektrische Folie (13) bilden, die auf Ebene der Rille (10), die mit einem elastischen Verbundmaterial (12) gefüllt ist, befestigt ist.

19. Messvorrichtung nach einem der Ansprüche 11 bis 13, **dadurch gekennzeichnet, dass** die Sonde (2) in dem zylindrischen Körper (4) eine Aussparung (14) aufweist, die die Platzierung und das Halten einer elektronischen Karte (15) erlaubt.

20. Messvorrichtung nach einem der Ansprüche 11 bis 13, **dadurch gekennzeichnet, dass** die Sonde (2) alle Sensoren aufweist, die für vaginale physiologische Messungen notwendig sind, die mit der elektronischen Karte (15) verbunden sind.

21. Messvorrichtung nach einem der Ansprüche 11 bis 13, **dadurch gekennzeichnet, dass** die elektronische Karte (15) an ein Gehäuse anschließbar ist, das einen oder Verstärker, einen A/D-Konverter und einen Mikroprozessor enthält.
